# FASCICULE DE BREVET EUROPEEN

(11) **EP 4 182 004 B1**
(45) Date de publication et mention de la délivrance du brevet: **22.04.2026**
(21) Numéro de dépôt: 21745774.6
(22) Date de dépôt: 15.07.2021
(51) Int. Cl.: A61M 60/178, A61M 60/422, A61M 60/232, A61M 60/82, A61M 60/857, A61M 60/861, A61M 60/822, A61M 60/833, A61B 17/12

(54) **SYSTÈME DE POMPE À SANG DE DÉCHARGEMENT ET SA POMPE À SANG**
ENTLADENDES BLUTPUMPENSYSTEM UND BLUTPUMPE DAFÜR
UNLOADING BLOOD PUMP SYSTEM AND THE BLOOD PUMP THEREOF

(30) Priorité: 16.07.2020 FR 2007445
(43) Date de publication de la demande: 24.05.2023
(73) Titulaire: ASSISTANCE PUBLIQUE HOPITAUX DE PARIS, 75004 Paris (FR)
(72) Inventeur: DANIAL, Pichoy, 94700 Maisons-Alfort (FR)
(74) Mandataire: Cabinet Camus Lebkiri
(86) Numéro de dépôt international: PCT/EP2021/069750
(87) Numéro de publication internationale: WO 2022/013349

(56) Documents cités:
- WO-A2-2005/037345
- WO-A2-2014/028787
- US-A1- 2003 069 468
- US-A1- 2020 093 972
- US-B1- 6 439 845

## Description

### DOMAINE TECHNIQUE DE L'INVENTION

Le domaine technique de l'invention se rapporte généralement au domaine des pompes à sang. Plus précisément, l'invention concerne une pompe à sang, adaptées à l'implantation chez l'homme et à décharger une cavité cardiaque pour diminuer le risque d'insuffisance cardiaque (IC) à fraction d'éjection préservée (ICFEP).

La présente invention concerne aussi un système de pompe à sang de déchargement d'une zone d'un cœur pour la réduction d'insuffisance cardiaque (IC) à fraction d'éjection préservée (ICFEP) et sa pompe à sang.

### ARRIERE-PLAN TECHNOLOGIQUE DE L'INVENTION

Il est connu comme maladie des insuffisances cardiaque (IC) qui peuvent être à fraction d'éjection préservée (ICFEP) ou peuvent être à fraction d'éjection réduite ou diminuée (ICFER). La distinction entre insuffisance à fraction d'éjection réduite ou diminuée (ICFER) et insuffisance cardiaque à fraction d'éjection préservée (ICFEP) repose notamment sur l'échocardiographie Doppler qui donne une valeur chiffrée de la fraction d'éjection du ventricule gauche (FEVG). Une fraction d'éjection FE < 40 - 50 % affirme l'IC à fraction d'éjection réduite ou diminuée (ICFER). Sinon, en cas de FE > 40 - 50 %, on évoque l'insuffisance cardiaque à fraction d'éjection préservée (ICFEP). Des critères précis de trouble de la relaxation et de dysfonction diastolique peuvent affirmer le diagnostic.

Il est connu des traitements pharmacologique et non pharmacologique ou des traitements chirurgicaux par dispositifs intracardiaques (stimulation bi ventriculaire) pour les insuffisances cardiaques mais aussi des dispositifs d'assistance ventriculaire pour les cas les plus important de réduction d'insuffisance cardiaque à fraction d'éjection réduite ou diminuée (ICFER). Les dispositifs d'assistance ventriculaire comprennent une pompe permettant d'assister le cœur en pompant du sang selon un débit selon le besoin du cœur dont le débit nominal utile est environ pour un adulte de 5l/min au repos et au cours d'un effort peut monter jusqu'à 35-40 L/min chez les sportifs. Ces pompes permettent d'assister le cœur entre 2L et 10L/min lorsque celui-ci ne permet pas d'avoir un débit cardiaque suffisant. Ces pompes sont assez volumineuses du fait de la puissance nécessaire pour pomper le débit de sang du ventricule gauche à l'aorte. Un exemple est présenté dans le document WO 2005/037345.

Les pompes à sang rotatives peuvent être centrifuges ou axiales. Dans une pompe à sang centrifuge, le sang entre dans la pompe le long de son axe de rotation et sort de la pompe perpendiculairement à l'axe de rotation. Dans une pompe à sang axiale, le sang entre dans la pompe le long de son axe de rotation et sort de la pompe le long de l'axe de rotation.

Notamment, il existe des pompes axiales d'assistances qui ont une vitesse de rotation très importantes, dont le rotor tourne par exemple à 32000 tours par minute pour assurer un débit de 2,5 L/min. Cette haute vitesse de rotation peut entrainer une thrombose du sang. Même si une telle pompe peut être réglée pour un débit plus faible par exemple 0.3l/min, sa vitesse de rotation sera très élevée de l'ordre de 17000 tours par minute et peut entraîner des risques d'hémolyses et de thromboses importants.

Les pompes axiales sont moins encombrantes que les pompes centrifuges à iso débit mais la pompe centrifuge permet un débit plus important à iso vitesse de rotation permettant de diminuer les problèmes de thromboses et d'hémolyses.

Cependant malgré de nombreux essais, aucun traitement ni pharmacologique ni chirurgicaux n'a fait la preuve de son efficacité dans l'insuffisance cardiaque à fraction d'éjection préservée (ICFEP). De ce fait, l'insuffisance cardiaque à fraction d'éjection préservée entraine beaucoup de décès, en France 30 à 50% des cas d'insuffisance cardiaque IC sont des insuffisances cardiaques à fraction d'éjection préservée (ICFEP) entraînant selon une étude américaine un taux de mortalité d'environ 29% à 1 an et d'environ 65% à 5 ans.

Il existe donc un besoin d'un traitement contre l'insuffisance cardiaque à fraction d'éjection préservée (ICFEP).

### RESUME DE L'INVENTION

L'invention offre une solution aux problèmes évoqués précédemment, en permettant de diminuer la pression atrio-pulmonaire sans augmenter le débit pulmonaire par rapport à la création d'un inter-atrial. En effet le retentissement pulmonaire de l'ICFEP est en rapport direct avec l'augmentation de pression et la dilatation passive des vaisseaux pulmonaires. Toute augmentation de pression entraîne une augmentation de diamètre de l'artère pulmonaire entrainant une diminution d'un cisaillement endothélial procurant des effets néfastes sur les fonctions endothéliales. Si le stimulus de pression perdure, un remodelage fonctionnel et structural se met en place dans la circulation artérielle pulmonaire aboutissant à une hypertension artérielle pulmonaire et ainsi l'insuffisance cardiaque droite. Ainsi en diminuant la pression capillaire pulmonaire, les symptômes de l'insuffisance cardiaque à fraction d'éjection préservée (ICFEP) sont réduits.

Un aspect de l'invention concerne un système de pompe à sang de déchargement comprenant :
- une canule d'aspiration comprenant une entrée d'aspiration configurée pour se connecter à une oreillette ou un ventricule et une extrémité de sortie,
- une canule de réinjection de flux comprenant une extrémité d'entrée et une extrémité de reflux connectée à l'aorte ou une artère en aval de l'aorte notamment une artère sous clavière par exemple gauche,
- une pompe à sang de déchargement comprenant :
   - un corps de chambre de pompage permettant le pompage du sang lorsqu'il est activé,
   - un orifice d'entrée relié à l'extrémité de sortie de la canule d'aspiration, pour aspirer du sang de la canule d'aspiration vers le corps de la chambre de pompage et
   - un orifice de sortie relié à l'extrémité d'entrée de la canule de réinjection de flux pour expulser du sang de chambre de pompage vers une canule de réinjection,
   - la pompe étant configurée pour en fonction de son alimentation permettre un débit continu constant nominal situé entre 0,05L/min et 0.5L/min afin de diminuer une pression capillaire pulmonaire et/ou de l'atrium gauche et/ou du ventricule gauche,
- un clip de ligature configuré pour ligaturer en amont l'artère sous clavière par rapport à l'extrémité de reflux de la canule de réinjection.

Grâce à l'invention, la pompe peut pomper du sang pour diminuer la pression de l'oreillette gauche appelée aussi atrium gauche ou/et du ventricule gauche en déchargeant par le biais de la pompe du sang circulant dans l'oreillette gauche, c'est-à-dire qu'une partie du sang provenant des veines pulmonaires va à une artère sans passer par la valve aortique. Notamment dans le cas où le sang est prélevé en étant aspiré par la pompe dans l'oreillette gauche, ce sang prélevé passe de l'oreillette gauche à une artère en aval de l'aorte sans passer par la valve mitrale, le ventricule gauche et la valve aortique. Le faible débit continu constant, par exemple, 0,5l/min permet de décharger du sang dans la partie gauche du cœur pour diminuer la pression atrio-pulmonaire sans augmenter le débit cardiaque autant qu'une pompe d'assistance. En effet la pression dans l'oreillette gauche au repos dans l'ICFEP est située entre 20 et 30 mmHg, ainsi lors de l'utilisation de la pompe, on la règle à une vitesse de rotation pour obtenir le débit constant (calculé) souhaité pour diminuer la pression atrio-pulmonaire du patient selon son besoin. Une telle décharge de sang dans l'oreillette gauche ou le ventricule gauche permet de diminuer la pression capillaire ou dans le ventricule gauche ou l'oreillette gauche. Par exemple les paramètres cliniques et échographiques permettent de savoir ensuite si le débit continu de la pompe est suffisant pour décharger le cœur suffisamment ou excessivement. Par exemple, on fait tourner la pompe pour décharger 0.05l/m par exemple à 2000 tours par minute, si la pression dans le cœur est toujours trop élevée (pression télédiastolique du ventricule gauche), on peut augmenter la vitesse de rotation et ainsi le débit jusqu'à obtenir la pression désirée, et vice-versa. En outre, une telle pompe est beaucoup moins puissante que les pompes d'assistances et est donc beaucoup moins volumineuse et demande en outre moins d'énergie. En effet, plus la pompe à un faible débit, moins elle est puissante et plus la taille est réduite ainsi que sa consommation d'énergie, par exemple électrique. Cela permet par exemple dans le cas de consommation d'énergie électrique de pouvoir avoir un moyen de stockage de batterie ou encore d'avoir un câble d'alimentation (pneumatique ou électrique) de plus petite taille que celle des pompes de l'art antérieur permettant ainsi de diminuer les risques d'infections. En outre le fait d'avoir un débit continu (sans accélération et décélération) permet de diminuer l'usure et augmente le rendement énergétique. Un autre avantage est que la section de l'entrée et de sortie de la partie pompe raccordé aux canules peut être plus petite du fait du débit moins important que celle des pompes à assistances. Une telle pompe permet ainsi de ne pas être surdimensionnée par rapport à celle de l'art antérieur dimensionnée pour produire un débit d'au moins 2L/min. Le clip permet de ligaturer afin d'éviter tout compétition de flux avec le cœur et permettre la décharge du cœur. En effet, le clip par exemple ligature à 90% de l'artère en amont de l'orifice de sortie pour éviter un risque de thrombose et d'événements thrombo-emboliques. Ceci évite des événements thrombo-emboliques dû à une compétition de flux possible entre le débit du cœur au niveau de l'artère sous-clavière et le débit de la pompe à sang de déchargement. Ainsi, clipper l'artère sous clavière en amont de la zone d'implantation de la canule de réinjection évite ces compétitions de flux.

Outre les caractéristiques qui viennent d'être évoquées dans les paragraphes précédents, le système de pompe à sang de déchargement selon un aspect de l'invention peut présenter une ou plusieurs caractéristiques complémentaires parmi les suivantes, considérées individuellement ou selon toutes les combinaisons techniquement possibles :

Selon un mode de réalisation, la pompe comprend un carter adapté à être incorporé dans un corps humain.

Dans le domaine d'implantation incorporelle, une telle pompe peut avoir une taille d'environ un pacemaker ce qui diminue la gêne d'un patient mais aussi réduit fortement la taille de la batterie d'énergie (pneumatique ou électrique) qui peut être plus facilement portable. Enfin, plus la consommation d'énergie (électrique au pneumatique) est faible plus la section des câbles d'alimentations d'énergie (électrique au pneumatique) de la partie moteur de la pompe peut être diminué.

Selon un mode de réalisation, le rotor est configuré pour tourner selon une vitesse continue entre 2000 et 5000 tours par minute et notamment entre 2000 tours par minute pour un débit de 0.05L/min et 5000 tours par minute pour un débit de 0.5 L/min. Une telle pompe permet de répondre à un besoin d'une majorité de patient et permet de réaliser la pompe à grande échelle. Ainsi, une telle pompe permet d'être encore plus petite.

Selon un mode de réalisation, la pompe comprend un débit maximum de 0.7l/min. Une telle pompe de déchargement aura donc une taille, une puissance et une consommation très inférieures à celle d'une pompe d'assistance.

Selon un mode de réalisation, la pompe à sang de déchargement comprend:
- un carter adapté à être incorporé dans un corps humain,
- au moins un stator solidaire du carter comprenant des bobines d'enroulements,
- un rotor monté centré et mobile en rotation par rapport au stator,
- une turbine tournée par le rotor,
- un connecteur d'alimentation électrique relié électriquement aux bobines d'enroulement,
- le corps de chambre de pompage étant montée dans le carter logeant la turbine,
- dans lequel l'orifice d'entrée permet d'aspirer du sang de la canule d'aspiration vers le corps de la chambre de pompage lorsque le rotor tourne la turbine et l'orifice de sortie permet d'expulser du sang de chambre de pompage vers la canule de réinjection.

Selon un exemple de ce mode de réalisation, la pompe est une pompe centrifuge. Cela permet de diminuer l'hémolyse par rapport à une pompe axiale et réduit l'usure de la pompe. De plus, les pompes axiales causent plus d'événements thrombo-emboliques que les pompes centrifuges.

Selon un exemple de ce mode de réalisation, le rotor est un rotor à aimants et le stator comprend un bobinage alimenté électriquement. Un tel rotor permet d'éviter d'avoir des balais encombrants et non permanent contrairement à des aimants.

Selon un exemple de ce mode de réalisation, la partie pompe est directement couplée à l'arbre rotor. Par directement couplé on entend qu'un tour de rotor est égal à un tour de la partie pompe.

Selon un exemple de ce mode de réalisation, le rotor forme une turbine rotor à pompe centrifuge logé dans le corps de la chambre de pompage pour pomper du sang à un débit compris entre 0.05 L/min et 0.5 L/min. La turbine rotor appelée aussi roue à aube ou impulseur permet lorsqu'elle est en rotation d'aspirer et d'éjecter le flux de liquide en l'occurrence le sang dans le corps de la chambre de pompe qui éjecte par l'orifice de sortie ce liquide.

Par exemple, le rotor comprend :
- un orifice axial en vis-à-vis de l'orifice d'entrée située axialement par rapport à l'axe de rotation du rotor,
- des aimants régulièrement répartis angulairement autour de l'orifice axiale,
- des encoches situées chacune entre deux aimants, chaque encoche s'étendant longitudinalement en débouchant de l'orifice d'entrée à une périphérie externe du rotor et ayant une rampe inclinée augmentant la profondeur axiale de l'encoche de l'orifice d'entrée vers la périphérie externe du rotor, et en ce que chaque encoche est ouverte sur le volume de la chambre de pompage entourant au moins les encoches du rotor,
- et le rotor formant la roue rotative située dans le corps de la chambre de pompage est adaptée pour pressuriser le sang rentrant par l'orifice d'entrée axialement et le sortir radialement par l'orifice de sortie en passant par la volute.

Selon un exemple de ce mode de réalisation, la pompe comprend une partie entraînement comprend plusieurs stators de moteur magnétique et le rotor a plusieurs régions magnétiques et en lévitation axialement et radialement en rotation par des forces magnétiques créées par des sources passives et actives de flux magnétique agissant sur le rotor et un ou plusieurs paliers de poussée hydrodynamiques prévus sur une surface supérieure de la roue.

Selon un exemple de ce mode de réalisation, la pompe est une pompe centrifuge à lévitation comprenant un palier passif à aimants permanents. Par exemple, le palier passif à aimants permanents comprend une pile d'aimants centrée sur l'axe fixée au stator, empilée en ayant une polarité Nord Sud alternée et une autre pile d'aimants fixée au rotor, entourant la première pile du stator pour exercer ensemble une première force axiale sur le rotor. Un tel palier permet d'une part de réduire les frottements et d'autre part de réduire la taille d'un palier à lévitation.

Selon un exemple de ce mode de réalisation, la pompe comprend une batterie reliée électriquement à la partie entrainement. Cela permet de pouvoir fonctionner sans être relié en permanence à une alimentation extérieure.

Par exemple, la pompe comprend un chargeur sans fil, de type inductif pour charger la batterie. Une telle pompe permet d'éviter d'avoir des câbles d'alimentation traversant la peau reliée à une batterie ou un chargeur. Selon cet exemple, la pompe comprend une unité de commande pour commander l'alimentation de la partie entrainante, notamment le stator. Une telle pompe permet d'éviter les problèmes d'infections des sorties de câble d'alimentation traversant la peau. Du fait de sa faible consommation, une telle pompe permet d'éviter les câbles d'alimentations traversant la peau pouvant entrainer des infections.

Selon une variante de l'exemple du mode de réalisation précédent, la pompe comprend un câble d'alimentation relié au connecteur, le câble étant adapté à passer dans un espace inter-costal, ou à travers la paroi abdominale, ou en rétro-auriculaire.

Selon un exemple de ce mode de réalisation, la pompe est à suspension magnétique et hydromécanique.

Selon un exemple de ce mode de réalisation, le carter de la pompe a une taille comprise entre 3 cm et 5 cm de diamètre et entre 1,5 cm et 4 cm de longueur axiale, en particulier 4 cm (diamètre) x 3 cm (longueur axiale). Une telle taille de pompe permet d'être implanter dans la région sous claviculaire par exemple sur ou sous le muscle grand pectoral, dans une poche réalisée dans la région sous claviculaire. Ainsi une telle pompe aura un carter ayant un diamètre externe plus petit qu'un diamètre de rotor à centrifuge d'une pompe à assistance qui est environ de 65mm avec une longueur axiale de 45mm. Autrement dit une telle pompe à déchargement est beaucoup plus petite que celle d'une pompe d'assistance.

Selon un mode de réalisation, la pompe est une pompe à déplacement positif, appelée aussi pompe volumétrique, dans laquelle par exemple la turbine comprend deux lobes pour le déplacement du sang.

Selon un exemple, la pompe volumétrique, est une pompe pneumatique ou électrique.

Selon un mode de réalisation, la canule d'aspiration comprend au moins une partie en polyéthylène téréphtalate sous forme textile (PET) ou en polytétrafluoroéthylène sous forme microporeuse (ePTFE) comprenant l'orifice proximale ou encore dans un autre matériau biocompatible.

Selon un exemple de ce mode de réalisation, la canule d'aspiration comprend une partie en titanium biocompatible traité comprenant l'extrémité d'aspiration et la partie en PET en ePTFE relie la partie en titanium à l'orifice d'entrée de la partie pompe.

Selon un mode de réalisation, la canule de réinjection comprend au moins une partie en PET ou ePTFE ou dans un autre matériau biocompatible comprenant l'orifice proximale.

Selon un exemple de ce mode de réalisation la canule de réinjection comprend une partie en titanium biocompatible traité comprenant l'extrémité d'entrée et la partie en PTFE relie la partie en titanium à l'orifice de sortie de la partie pompe.

Selon un mode de réalisation, la canule de réinjection et la canule d'aspiration ont un diamètre compris entre 5 mm et 10 mm.

Selon un mode de réalisation, le clip s'étend à partir d'une partie de la canule de réinjection proche de l'orifice de sortie. Cela permet lors de l'opération d'avoir le clip près de la canule et ainsi aider le chirurgien lors de l'opération.

Selon un mode de réalisation, la pompe est une pompe à alimentation électrique et le système comprend un dispositif de commande et d'alimentation comprenant une batterie et une unité de commande pour commander l'alimentation électrique de la pompe par la pompe à un débit continu prédéfini.

Selon un mode de réalisation, l'unité de commande est destinée à commander la pompe en continu (sans interruption). Cela permet d'éviter le risque de thrombose et d'événements thrombo-emboliques. En cas de batterie faiblement rechargée, le système peut comprendre un dispositif de signalement, par exemple sonore ou lumineux, commander par l'unité de commande en fonction de la tension de la batterie mesurée par l'unité de commande, pour avertir l'utilisateur.

Selon un mode de réalisation, le système comprend un câble d'alimentation relié à un connecteur de la pompe et à l'unité de commande, le câble d'alimentation étant configuré pour traverser la peau d'un corps humain.

Selon un mode de réalisation, le carter comprend une enveloppe qui s'étend de matière avec une portion de la canule de réinjection et de la canule d'aspiration.

Un autre aspect non revendiqué de la présente divulgation concerne une méthode d'implantation d'un système de pompe à sang de déchargement (par exemple celui selon l'aspect de l'invention décrit précédemment avec ou sans les différentes caractéristiques des modes de réalisation mentionnées précédemment), la méthode comprenant :
- Soit une première étape de clampage de l'atrium gauche puis une étape d'anastomose de l'extrémité d'aspiration de la canule d'aspiration directement sur l'atrium gauche puis une étape de retrait du clampage de l'atrium gauche, ou soit une étape de mise du ventricule gauche sous fibrillation ventriculaire puis une étape d'anastomose de l'extrémité d'aspiration de la canule d'aspiration dans la pointe du ventricule gauche sous fibrillation ventriculaire
- une étape de débullage de la pompe de décharge,
- une étape de ligature par exemple par clippage, de l'artère sous clavière pour ligaturer entre 85 et 100% de l'artère.
- une étape de clampage dans deux zones de l'artère sous clavière,
- une étape d'anastomose de la canule de réinjection en insérant son extrémité de reflux entre les deux clampages et en aval du clippage,
- une étape de retrait du clampage de l'artère sous clavière,
- une étape de calcul du débit à décharger du sang d'une oreillette gauche pour éviter un ICFEP
- une étape de réglage de l'unité de commande de la pompe selon le débit calculé,
- une étape de mise en route de la pompe par l'unité de commande.

Un autre aspect non revendiqué de la présente divulgation concerne une méthode d'implantation d'un système de pompe à sang de déchargement (par exemple celui selon l'aspect de l'invention décrit précédemment avec ou sans les différentes caractéristiques des modes de réalisation mentionnées précédemment), la méthode comprenant :
- une étape d'insertion de l'extrémité d'aspiration de la canule d'aspiration dans l'atrium gauche, par insertion de la canule d'aspiration par voie endovasculaire à travers d'une part la veine jugulaire interne puis l'oreillette droite et enfin à travers le septum inter atrial pour rentrer dans l'atrium gauche,
- une étape de débullage de la pompe de décharge,
- une étape de ligature par exemple par clippage, de l'artère sous clavière pour ligaturer entre 85 et 100% de l'artère.
- une étape de clampage dans deux zones de l'artère sous clavière,
- une étape d'anastomose de la canule de réinjection en insérant son extrémité de reflux entre les deux clampages et en aval du clippage,
- une étape de retrait du clampage de l'artère sous clavière,
- une étape de calcul du débit à décharger du sang d'une oreillette gauche pour éviter un ICFEP
- une étape de réglage de l'unité de commande de la pompe selon le débit calculé,
- une étape de mise en route de la pompe par l'unité de commande.

Un autre aspect de l'invention non revendiqué concerne une pompe à sang de déchargement comprenant :
- un carter adapté à être incorporé dans un corps humain,
- au moins un stator solidaire du carter comprenant des bobines d'enroulements,
- un rotor monté centré et mobile en rotation par rapport au stator,
- une turbine tournée par le rotor,
- un connecteur d'alimentation électrique relié électriquement aux bobines d'enroulement,
- un corps de chambre de pompage montée dans le carter logeant la turbine,
- un orifice d'entrée pour aspirer du sang d'une canule d'aspiration vers le corps de la chambre de pompage lorsque le rotor tourne la turbine et un orifice de sortie pour expulser du sang de chambre de pompage vers une canule de réinjection,
- caractérisé en ce que la pompe est configurée pour en fonction de son alimentation permettre un débit continu constant nominal situé entre 0,05L/min et 0.5L/min afin de diminuer une pression capillaire pulmonaire, et/ou de l'atrium gauche et/ou du ventricule gauche.

La pompe peut comprendre les caractéristiques décrites dans les exemples du mode de réalisation du système de pompe selon l'aspect de l'invention décrit précédemment comprenant une telle pompe à sang.

Un autre aspect de l'invention non revendiqué concerne une pompe à sang de déchargement comprenant :
- un carter adapté à être incorporé dans un corps humain,
- une membrane,
- un connecteur d'alimentation pneumatique pour déplacer la membrane,
- un corps de chambre de pompage monté dans le carter logeant la membrane,
- un orifice d'entrée pour aspirer du sang d'une canule d'aspiration vers le corps de la chambre de pompage lorsque le rotor tourne la turbine et un orifice de sortie pour expulser du sang de chambre de pompage vers une canule de réinjection,

caractérisé en ce que la pompe est configurée pour en fonction de son alimentation permettre un débit continu constant nominal situé entre 0,05L/min et 0.5L/min afin de diminuer une pression capillaire pulmonaire, et/ou de l'atrium gauche et/ou du ventricule gauche.

L'invention et ses différentes applications seront mieux comprises à la lecture de la description qui suit et à l'examen des figures qui l'accompagnent. L'invention est définie par la revendication 1.

### BREVE DESCRIPTION DES FIGURES

Les figures sont présentées à titre indicatif et nullement limitatif de l'invention.
[Fig. 1] représente un schéma de principe d'un système de pompe à sang de déchargement selon l'invention implantée pour diminuer une pression capillaire selon une première utilisation,
[Fig. 2] représente selon un schéma de principe d'une pompe à sang de déchargement du système selon un premier mode de l'invention.
[Fig. 3] représente un schéma de principe d'un système de pompe à sang de déchargement selon l'invention implantée pour diminuer une pression capillaire selon une deuxième utilisation

### DESCRIPTION DETAILLEE

Les figures sont présentées à titre indicatif et nullement limitatif de l'invention.

Par débit continu on entend un débit par la pompe + ou - 10% son débit.

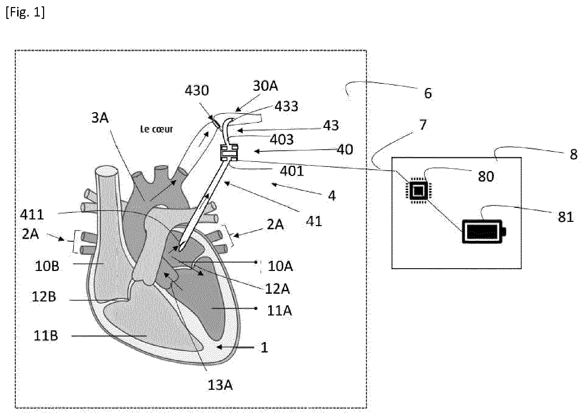

La figure 1 montre un schéma de principe d'un schéma de principe d'un système de pompe à sang de déchargement selon l'invention implantée pour diminuer une pression capillaire selon une première utilisation.

Le patient comprend un cœur 1 comprenant une partie gauche A et une partie droite B. Des vaisseaux sanguins 2A alimentent en sang la partie gauche du cœur qui l'aspire et refoule le sang dans une aorte 3A. La partie gauche A comprend une oreillette gauche 10A, un ventricule gauche 11A, une valve auriculoventriculaire gauche appelée aussi valve mitrale 12A entre l'oreillette gauche 10A et le ventricule gauche 11A, et une valve aortique 13A entre le ventricule gauche 11A et l'aorte 3A. L'aorte 3A distribue le sang dans différentes artères dont l'artère sous-clavière gauche 30A. La partie droite B comprend aussi une oreillette droite 10B, un ventricule droit 11B, une valve auriculoventriculaire droite appelée aussi valve tricuspide 12B entre l'oreillette droite 10B et le ventricule droit 11B. Des flèches représentent la circulation du sang 3A dans le cœur 1 provenant des vaisseaux sanguins 2A en passant d'abord par oreillette gauche 10A, ensuite par la valve auriculoventriculaire 12A dans le ventricule gauche 11A et enfin dans l'aorte 3A par le biais de la valve aortique 13A.

Le système de pompe à sang de déchargement 4 est monté dans cette première utilisation pour décharger du sang de l'oreillette gauche 10A à l'artère sous-clavière gauche 30A.

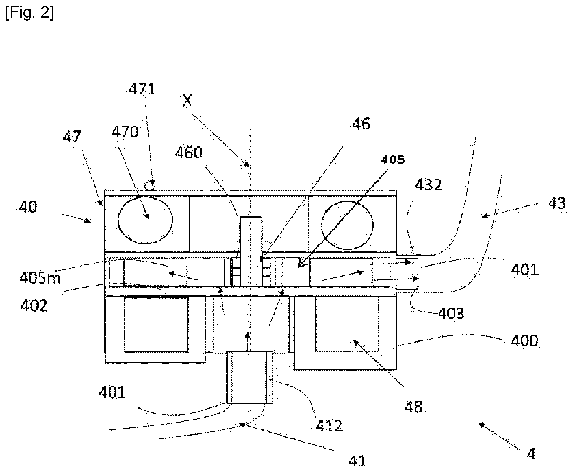

Le système de pompe à sang de déchargement 4 comprend une canule d'aspiration 41, une canule de réinjection 43 et une pompe à sang de déchargement 40 et dans cet exemple de ce mode de réalisation un organe de ligature en l'occurrence un clip 430 qui peut être remplacé par un lasso. La pompe à sang 40 comprend un orifice d'entrée 401, raccordé à une extrémité de sortie 412 (référencée sur la figure 2) de la canule d'aspiration 41 et un orifice de sortie 403 raccordé à une extrémité d'entrée 432 (référencée sur la figure 2) de la canule de réinjection 43. La canule d'aspiration 41 comprend une extrémité d'aspiration 411, opposée à l'extrémité de sortie 412, qui dans cet exemple est située dans l'oreillette gauche 11A et la canule de réinjection 43 comprend une extrémité de reflux 433 opposée à l'extrémité d'entrée 434 située dans cet exemple dans l'artère sous-clavière gauche. Le clip 430 est clipsé sur l'artère sous clavière gauche en amont de l'extrémité de reflux 433, pour ligaturer l'artère. Dans l'exemple d'un lasso, le lasso est enroulé sur l'artère sous clavière gauche en amont de l'extrémité de reflux 433. Le clip 430 ou le lasso peut être adapté à ligaturer entre 85% et 100% une artère sous clavière gauche. Le clip 430 ou le lasso est en l'occurrence lié à la canule de réinjection 43 permettant ainsi d'améliorer l'opération chirurgicale. La canule de réinjection 43 et la canule d'aspiration 41 ont chacun un diamètre interne compris entre 5mm et 10mm, par exemple 5mm pour la canule d'aspiration 41 et 8mm pour la canule de réinjection 43. Les canules 41, 43 sont en l'occurrence en polyéthylène téréphtalate sous forme textile (PET) ou en polytétrafluoroéthylène sous forme microporeuse (ePTFE) ou encore dans un autre matériau biocompatible

La pompe à sang de déchargement 40 permet donc d'aspirer du sang dans l'oreillette gauche 10A par l'extrémité d'aspiration 411 de la canule d'aspiration 41 et de le rejeter dans l'artère sous clavière 30A par l'extrémité de reflux 433 de la canule de réinjection 43. La pompe à sang de déchargement 40 est configurée pour absorber un débit de sang entre 0.05 litre par minute et 0.5 litre par minute. Ce débit permet ainsi de décharger la pression dans l'oreillette gauche et donc ainsi aussi la pression capillaire pulmonaire.

La figure 2 représente un schéma de principe de la pompe à sang de déchargement 40 reliée à l'extrémité de sortie 412 de la canule d'aspiration 41 et à l'extrémité d'entrée 432 de la canule de refoulement 43.

La pompe à sang de déchargement 40 comprend un carter 400 adapté à être incorporé dans un corps humain 6 dans lequel est située un corps de chambre de pompage 402 étanche en forme de volute.

Le corps de chambre de pompage 402 comprend, dans cet exemple, un volume de 4 à 10 millilitres et est ouverte sur l'orifice d'entrée 401 ayant un diamètre interne dans cet exemple supérieur à l'extrémité de sortie 412 de la canule d'aspiration 41, par exemple un diamètre d'1 mm de plus que celle de l'extrémité de sortie 412 soit dans cet exemple 6 mm. Le corps de la chambre de pompage 402 est en outre ouverte sur une extrémité de la forme volute sur l'orifice de sortie 403 ayant un diamètre interne, dans cet exemple, supérieur d'1 mm à celle de la canule de réinjection 43 soit ici 9 mm. Le corps de chambre de pompage 402 est en l'occurrence en titanium biocompatible.

La pompe à sang de déchargement 40 comprend une roue turbine logée dans le corps de chambre de pompage 402.

La pompe à sang de déchargement 40 comprend un moteur électrique formant la partie entrainante de la pompe à sang de déchargement 40. En l'occurrence la pompe à sang de déchargement 40 est une pompe à centrifuge électrique. En particulier, la roue turbine est aussi le rotor du moteur électrique ayant un axe de rotation X.

La roue turbine est donc dans cet exemple une turbine rotor 405 comprenant un corps turbine et des aimants permanents ferromagnétique 405m logés dans le corps. Les aimants peuvent par exemple être en néodyme ou en alliage Alnico ou encore en alliage cobalt platine. En outre, la turbine rotor 405 comprend par exemple une couche de polymère (Parylène et silicone), par exemple un surmoulage en polymère sur les aimants. La couche peut en outre être traité biocompatible par nitrure de chrome ou/et nitrure de titane. La roue turbine 405 peut ainsi comprendre, par exemple, quatre aimants répartis angulairement dans quatre logements du corps autour de l'axe de rotation X, et sont positionnés en ayant leurs polarités de façon alternée angulairement. La turbine rotor comprend des encoches situées chacune entre deux aimants, chaque encoche s'étendant longitudinalement en débouchant de l'orifice d'entrée 412 à une périphérie externe de la turbine rotor. L'encoche peut comprendre une rampe inclinée augmentant la profondeur axiale de l'encoche de l'orifice d'entrée vers la périphérie externe du rotor, et en ce que chaque encoche est ouverte sur le volume de la chambre de pompage entourant au moins les encoches de la turbine rotor. Des flèches sur la figure représentent ainsi la circulation du sang dans la turbine rotor.

Le corps de la turbine rotor 405 a, dans cet exemple, un diamètre externe entre 15 et 30 millimètres avec une longueur axiale appelée aussi hauteur axiale comprise entre 5 et 20 millimètres et le carter 400 dans cet exemple a une taille environ de 40x30mm pour y loger le ou les stators du moteur électriques en plus du corps de chambre de pompage. Du fait du faible débit, la pompe à sang de déchargement 40 est ainsi beaucoup plus petite qu'une pompe d'assistance de l'art antérieur et comprend notamment un diamètre compris entre 30mm et 50mm et une longueur axiale comprise entre 15mm et 40mm, par exemple dans cet exemple : 40mm de diamètre et 30mm de longueur axiale.

Le corps de turbine est en Titanium biocompatible, par exemple nitrite de titane ou encore en matériau céramique.

Ainsi, un tel corps de chambre de pompage 402 avec une telle turbine rotor 405 peut permettre à la pompe de débiter entre 0.05L/min et 0.5L/min de sang en tournant à une vitesse de rotation entre 2000 et 5000 tours par minute.

Le moteur électrique de la pompe à sang de déchargement 40 comprend un ou plusieurs stators logés dans le carter, dont au moins un stator bobiné 47 comprend des bobines d'enroulement 470 produisant un champ magnétique lorsqu'elles sont alimentées électriquement pour produire un couple de rotation avec la turbine rotor 405. Le ou l'autre stator peuvent comprend des aimants permanents. La pompe à sang de déchargement 40 comprend en outre un connecteur d'alimentation électrique 471 relié électriquement aux bobines d'enroulement 470 et le système comprend en outre un câble d'alimentation 7 relié au connecteur 471 de la pompe et à un dispositif de commande et d'alimentation 8. Le connecteur 471 peut comprendre un connecteur électrique en matériau conducteur tel que le platine ou en cuivre et est recouvert d'un isolant tel que du PolyEtherEtherKeton (PEEK) ou Polysulfone (PSU) ou encore un époxy de qualité médical.

Dans cet exemple, le dispositif de commande et d'alimentation 8 est extra corporel. Le câble d'alimentation 7 est donc configuré pour traverser la peau d'un corps humain 6, en particulier ici, à passer dans un espace inter-costal ou à travers la paroi abdominale ou en rétro-auriculaire. Le câble d'alimentation 7 peut aussi être en platine et être recouvert d'un isolant médical tel que celui des canules ou du connecteur 471.

Le dispositif de commande et d'alimentation 8 comprend une unité de commande 80 pour commander la puissance transmise au moteur de la pompe et donc sa la vitesse de rotation et le débit de la pompe à sang. Le dispositif de commande et d'alimentation 8 comprend en outre une batterie 81 alimentant électriquement les bobines d'enroulement 470 du stator 47 de la pompe à sang 40.

Dans cet exemple, le moteur électrique comprend deux stators, un premier stator inférieur 48 entourant l'orifice d'entré comprenant des aimants 480, par exemple au nombre de quatre régulièrement répartis autour de l'axe de rotation et l'autre stator bobiné supérieur 47 à l'opposé axialement du stator inférieur par rapport à la turbine rotor 405. Le stator bobiné 47 est plus proche axialement que le stator inférieur 48 exerçant ainsi une force axiale supérieure. Les deux stators comprennent un diamètre de 15mm avec une hauteur de 8mm.

En particulier dans cet exemple, la pompe à sang de déchargement 40 est une pompe centrifuge électrique à lévitation comprenant un palier passif à aimants permanents 46. Le palier à lévitation 46 comprend un axe s'étendant du stator supérieur 47 axialement dans le corps de chambre de pompage 402 et comprend une pluralité d'aimants permanent interne 460 solidaire de l'axe, par exemple 3 aimants, empilés axialement en ayant leurs deux pôles répétés de façon identique NS/NS/NS. Le palier à lévitation comprend en outre une pluralité d'aimants permanent externe cylindrique creux 461 montés sur le corps de turbine rotor 405 et empilés axialement en ayant leurs deux pôles répétés de façon identique NS/NS/NS entourant les aimants permanent internes 460. Ce palier à lévitation permet de produire un centrage de la pompe radiale de façon répulsive avec les aimants et en outre par le biais de l'attraction axiale du stator supérieur 47.

Une telle pompe à sang de déchargement 40 permet de fonctionner de façon continue avec peu d'usure tout en ayant un débit de sang compris entre 0.05 L/min et 0.5 L/min. Ainsi dans certain cas en fonction de la formation du cœur du patient, une pompe à sang peut être adapté à avoir un débit entre 0.05l/min et 0.3l/min et donc être encore plus petite, par exemple comprendre un carter de diamètre de 3cm et de longueur axiale de 1.5cm et un corps de la turbine rotor de diamètre de 15 millimètres et de longueur axiale de 5 millimètres. Le débit continu de la pompe est réglé en fonction des caractéristiques du cœur et de la dépression à effectuer.

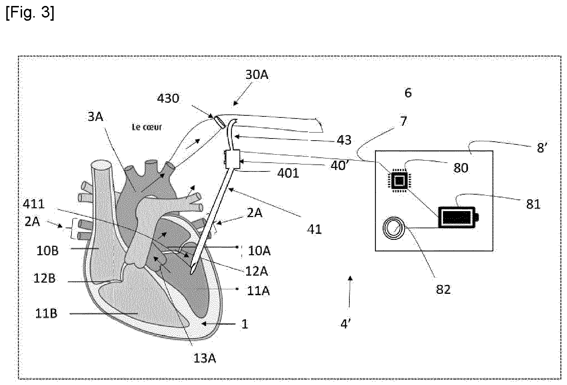

La figure 3 représente une deuxième utilisation d'un autre exemple de système de pompe à sang de déchargement 4' pompe selon le premier mode de réalisation.

Ce système de pompe à sang de déchargement 4' est identique au premier exemple sauf en ce que la canule d'aspiration 41 et la canule de refoulement 43 sont fait d'une seule matière et entoure le carter de la pompe 40 et en ce que le dispositif de commande et d'alimentation 8' est adapté à être incorporel et comprend un chargeur à induction 82 pour charger la batterie 81. Ce dispositif de commande et d'alimentation 8' peut être positionné dans un autre espace du corps tel que par exemple dans la paroi thoracique, sous un muscle grand pectoral ou sous un muscle grand dorsal. La deuxième utilisation est identique à la première utilisation sauf en ce que la canule d'aspiration 41 traverse le ventricule gauche 11A et donc comprend son extrémité d'aspiration 411 dans ce ventricule gauche pour aspirer du sang et le refouler dans l'artère sous clavicule gauche 30A.

La méthode d'implantation du système de pompe à sang de déchargement 4 comprend dans la première utilisation deux modes de réalisation. Dans le premier mode de réalisation, la méthode comprend une première étape de clampage de l'atrium gauche 10A et ensuite une étape d'anastomose de l'extrémité d'aspiration 411 de la canule d'aspiration 41 sur l'atrium gauche 10A puis une étape de retrait du clampage, de l'atrium gauche.

Dans le deuxième mode de réalisation de la première utilisation, la méthode comprend une première étape d'insertion de l'extrémité d'aspiration 411 de la canule d'aspiration 41 dans l'oreillette gauche 10A, par voie endovasculaire à travers dans un premier temps la veine jugulaire interne puis l'oreillette droite puis en traversant le septum inter atrial pour l'insertion de l'extrémité de la canule d'aspiration 411 dans l'oreillette gauche 10A.

Par étape d'anastomose d'une canule dans une partie du cœur, on entend l'insertion de l'extrémité de la canule dans la partie du cœur pour la connexion de cette canule avec le volume de cette partie du cœur.

La méthode d'implantation du système de pompe à sang de déchargement 4 comprend dans la deuxième utilisation une étape de mise du ventricule gauche sous fibrillation ventriculaire puis une étape d'anastomose de l'extrémité d'aspiration 411 de la canule d'aspiration 41 directement dans le ventricule gauche 11A.

Dans l'étape de mise du ventricule gauche sous fibrillation ventriculaire permet un arrêt de la contraction pendant quelques secondes le temps d'insérer la canule d'aspiration. L'étape de mise sous fibrillation ventriculaire peut comprendre une sous étape de mise en place de points et une collerette sur le ventricule gauche à cœur battant avant l'arrêt des contractions.

La méthode comprend ensuite dans les deux utilisations, une étape de débullage de la pompe de décharge.

La méthode comprend ensuite dans les deux utilisations, une étape de ligature par exemple par clippage du clip 430, de l'artère sous clavière 30A pour ligaturer entre 85 et 100% l'artère selon le débit calculé.

La méthode comprend ensuite, une étape de clampage dans deux zones de l'artère sous clavière 30A,

La méthode comprend ensuite, une étape d'anastomoser la canule de réinjection 43 en insérant son extrémité de reflux 433 entre les deux clampages et en aval du clippage 430,
une étape de retrait du clampage de l'artère sous clavière,
une étape de calcul du débit à décharger du sang d'une oreillette gauche pour éviter un ICFEP
une étape de réglage de l'unité de commande de la pompe selon le bit calculé,
une étape de mise en route de la pompe par l'unité de commande.

Sauf précision contraire, un même élément apparaissant sur des figures différentes présente une référence unique.

## Revendications

1. Système de pompe à sang de déchargement (4, 4') comprenant :
- une canule d'aspiration (41) comprenant une entrée d'aspiration (411) configurée pour se connecter à une oreillette (10A, 10B) ou un ventricule (11A, 11B) et une extrémité de sortie (412),
- une canule de réinjection (43) de flux comprenant une extrémité d'entrée (432) et une extrémité de reflux (433) configurée pour se connecter à l'aorte ou une artère en aval de l'aorte notamment une artère sous clavière par exemple gauche,
- une pompe à sang de déchargement (40, 40') comprenant :
∘ un corps de chambre de pompage (402) permettant le pompage du sang lorsqu'il est activé,
∘ un orifice d'entrée (401) relié à l'extrémité de sortie (412) de la canule d'aspiration (41), pour aspirer du sang de la canule d'aspiration (41) vers le corps de la chambre de pompage (402) et
∘ un orifice de sortie (403) relié à l'extrémité d'entrée (432) de la canule de réinjection (43) de flux pour expulser du sang de chambre de pompage (402) vers une canule de réinjection (43),
∘ la pompe (40, 40') étant configurée pour en fonction de son alimentation permettre un débit continu constant nominal situé entre 0,05L/min et 0.5L/min afin de diminuer une pression capillaire pulmonaire et/ou de l'atrium gauche et/ou du ventricule gauche,
- un clip de ligature (430) configuré pour ligaturer en amont l'artère sous clavière (30A) par rapport à l'extrémité de reflux (433) de la canule de réinjection (43).

2. Système de pompe à sang de déchargement (4, 4') selon la revendication 1 dans lequel la pompe à sang de déchargement (40, 40') comprend :
- un carter (400) adapté à être incorporé dans un corps humain (6),
- au moins un stator (47, 48) solidaire du carter comprenant des bobines d'enroulements (470),
- un rotor monté centré et mobile en rotation par rapport au stator (47, 48),
- une turbine tournée par le rotor,
- un connecteur d'alimentation électrique (471) relié électriquement aux bobines d'enroulement (470),
- le corps de chambre de pompage (402) étant montée dans le carter (400) logeant la turbine,
- dans lequel l'orifice d'entrée (401) permet d'aspirer du sang de la canule d'aspiration (41) vers le corps de la chambre de pompage (402) lorsque le rotor tourne la turbine et l'orifice de sortie (403) permet d'expulser du sang de chambre de pompage (402) vers la canule de réinjection (43).

3. Système de pompe à sang de déchargement (4, 4') selon la revendication 2 dans lequel la pompe à sang (40, 40') dans lequel le rotor est configuré pour tourner selon une vitesse continue entre 2000 et 5000 tours par minute et notamment entre 2000 tours par minute pour un débit de 0.05l/min et 5000 tours par minute pour un débit de 0.5l/min.

4. Système de pompe à sang de déchargement (4, 4') selon la revendication 2 ou 3, dans lequel la pompe est une pompe centrifuge.

5. Système de pompe à sang de déchargement (4, 4') selon l'une quelconques des revendications 2 à 4, dans lequel le rotor forme une turbine rotor (405) à pompe centrifuge logé dans le corps de la chambre de pompage (402) pour pomper du sang à un débit compris entre 0.05l/min et 0.5l/min.

6. Système de pompe à sang de déchargement (4, 4') selon l'une des revendications précédentes, dans lequel la pompe à sang comprend un débit maximum de 0.7 L/min

7. Système de pompe à sang de déchargement (4, 4') selon l'une des revendications précédentes, dans lequel la canule de réinjection (43) et la canule d'aspiration (41) ont un diamètre compris entre 5 et 10 mm.

8. Système de pompe à sang de déchargement (4, 4') selon l'une des revendications précédentes, dans lequel le clip s'étend à partir d'une partie de la canule de réinjection proche de l'orifice de sortie.

## Patentansprüche

1. Blutentleerungspumpensystem (4, 4') mit:
- einer Saugkanüle (41) mit einem Saugeinlass (411), der zur Verbindung mit einem Vorhof (10A, 10B) oder einem Ventrikel (11A, 11B) gestaltet ist, und einem Auslassende (412),
- einer Reinjektionskanüle (43) mit einem Einlassende (432) und einem Rückflussende (433), die so gestaltet ist, dass sie mit der Aorta oder einer Arterie stromabwärts der Aorta, insbesondere einer Arteria subclavia, beispielsweise der linken, verbunden werden kann,
- einer Blutentleerungspumpe (40, 40'), die umfasst:
∘ einen Pumpenkammerkörper (402), der das Pumpen von Blut ermöglicht, wenn er aktiviert ist,
∘ eine Einlassöffnung (401), die mit dem Auslassende (412) der Saugkanüle (41) verbunden ist, um Blut aus der Saugkanüle (41) in den Pumpenkammerkörper (402) zu saugen, und
∘ eine Auslassöffnung (403), die mit dem Einlassende (432) der Reinjektionskanüle (43) verbunden ist, um Blut aus der Pumpenkammer (402) in eine Reinjektionskanüle (43) zu drücken,
∘ wobei die Pumpe (40, 40') so gestaltet ist, dass sie entsprechend ihrer Speisung einen konstanten Nenndurchfluss zwischen 0,05 l/min und 0,5 I/min ermöglicht, um den Kapillardruck in der Lunge und/oder im linken Vorhof und/oder im linken Ventrikel zu senken,
- eine Ligaturklemme (430), die so gestaltet ist, dass sie die Arteria subclavia (30A) stromaufwärts in Bezug zum Rückflussende (433) der Reinjektionskanüle (43) abklemmt.

2. Blutentleerungspumpensystem (4, 4') gemäß Anspruch 1, in dem die Blutentleerungspumpe (40, 40') umfasst:
- ein Gehäuse (400), das zum Einbau in einen menschlichen Körper (6) geeignet ist,
- mindestens einen mit dem Gehäuse verbundenen Stator (47, 48) mit Wicklungsspulen (470),
- einen Rotor, der zentriert und drehbar in Bezug auf den Stator (47, 48) angebracht ist,
- eine vom Rotor angetriebene Turbine,
- einen Stromanschluss (471), der elektrisch mit den Wicklungsspulen (470) verbunden ist,
- wobei der Pumpenkammerkörper (402) in dem die Turbine aufnehmenden Gehäuse (400) angebracht ist,
- wobei die Einlassöffnung (401) das Ansaugen von Blut aus der Saugkanüle (41) in den Pumpenkammerkörper (402) ermöglicht, wenn der Rotor die Turbine dreht, und die Auslassöffnung (403) das Ausstoßen von Blut aus der Pumpenkammer (402) in die Reinjektionskanüle (43) ermöglicht.

3. Blutentleerungspumpensystem (4, 4') gemäß Anspruch 2, in dem die Blutpumpe (40, 40') so gestaltet ist, dass sie den Rotor mit einer kontinuierlichen Geschwindigkeit zwischen 2000 und 5000 Umdrehungen pro Minute dreht, insbesondere zwischen 2000 Umdrehungen pro Minute für einen Durchfluss von 0,05 I/min und 5000 Umdrehungen pro Minute für einen Durchfluss von 0,5 l/min.

4. Blutentleerungspumpensystem (4, 4') gemäß Anspruch 2 oder 3, in dem die Pumpe eine Zentrifugalpumpe ist.

5. Blutentleerungspumpensystem (4, 4') gemäß einem der Ansprüche 2 bis 4, bei der der Rotor eine Kreiselpumpenrotorturbine (405) bildet, die im Gehäuse der Pumpenkammer (402) untergebracht ist, um Blut mit einer Durchflussrate zwischen 0,05 I/min und 0,5 I/min zu pumpen.

6. Blutentleerungspumpensystem (4, 4') gemäß einem der vorstehenden Ansprüche, in dem die Blutpumpe eine maximale Durchflussrate von 0,7 I/min aufweist.

7. Blutentleerungspumpensystem (4, 4') gemäß einem der vorstehenden Ansprüche, in dem die Reinjektionskanüle (43) und die Saugkanüle (41) einen Durchmesser zwischen 5 und 10 mm aufweisen.

8. Blutentleerungspumpensystem (4, 4') gemäß einem der vorstehenden Ansprüche, in dem sich die Klammer ab einem Bereich der Reinjektionskanüle in der Nähe der Auslassöffnung erstreckt.

## Claims

1. A blood discharge pump system (4, 4') comprising:
• a suction cannula (41) comprising a suction inlet (411) configured to connect to an atrium (10A, 10B) or ventricle (11A, 11B) and an outlet end (412),
• a reflux cannula (43) comprising an inlet end (432) and a reflux end (433) configured to connect to the aorta or an artery downstream of the aorta, in particular a subclavian artery, for example the left subclavian artery,
• a blood discharge pump (40, 40') comprising:
• a pumping chamber body (402) for pumping blood when activated,
∘ an inlet port (401) connected to the outlet end (412) of the suction cannula (41) for sucking blood from the suction cannula (41) into the pumping chamber body (402) and
∘ an outlet port (403) connected to the inlet end (432) of the re-injection cannula (43) for expelling blood from the pumping chamber (402) to a reinjection cannula (43),
∘ the pump (40, 40') being configured to allow, depending on its power supply, a nominal continuous flow rate of between 0.05 L/min and 0.5 L/min in order to reduce pulmonary capillary pressure and/or left atrium pressure and/or left ventricle pressure,
• a ligation clip (430) configured to ligate the subclavian artery (30A) upstream of the reflux end (433) of the reinjection cannula (43).

2. A blood unloading pump system (4, 4') according to claim 1, wherein the blood unloading pump (40, 40') comprises:
• a casing (400) adapted to be incorporated into a human body (6),
• at least one stator (47, 48) integral with the casing comprising winding coils (470),
• a rotor mounted centrally and rotatably with respect to the stator (47, 48),
• a turbine rotated by the rotor,
• an electrical power connector (471) electrically connected to the winding coils (470),
• the pumping chamber body (402) being mounted in the casing (400) housing the turbine,
• wherein the inlet port (401) allows blood to be drawn from the suction cannula (41) into the pumping chamber body (402) when the rotor turns the turbine, and the outlet port (403) allows blood to be expelled from the pumping chamber (402) to the re-injection cannula (43).

3. A blood discharge pump system (4, 4') according to claim 2, wherein the blood pump (40, 40') in which the rotor is configured to rotate at a continuous speed between 2000 and 5000 revolutions per minute and in particular between 2000 revolutions per minute for a flow rate of 0.05 I/min and 5000 revolutions per minute for a flow rate of 0.5 I/min.

4. A blood discharge pump system (4, 4') according to claim 2 or 3, wherein the pump is a centrifugal pump.

5. A blood discharge pump system (4, 4') according to any of claims 2 to 4, wherein the rotor forms a centrifugal pump rotor turbine (405) housed in the pump chamber body (402) for pumping blood at a flow rate between 0.05 I/min and 0.5 I/min.

6. A blood discharge pump system (4, 4') according to any of the preceding claims, wherein the blood pump comprises a maximum flow rate of 0.7 L/min.

7. A blood discharge pump system (4, 4') according to any of the preceding claims, wherein the reflux cannula (43) and the suction cannula (41) have a diameter comprise between 5 and 10 mm.

8. A blood discharge pump system (4, 4') according to any of the preceding claims, wherein the clip extends from a part of the reflux cannula near the reflux end.
